# FASCICULE DE BREVET EUROPEEN

(11) **EP 2 204 163 B1**
(45) Date de publication et mention de la délivrance du brevet: **29.11.2017**
(21) Numéro de dépôt: 09181018.4
(22) Date de dépôt: 30.12.2009
(51) Int. Cl.: A61K 8/60, A61K 8/67, A61Q 19/08

(54) **Association de monosaccharides avec l'acide ascorbique et son utilisation en cosmétique**
Kombination aus Monosaccharide und Ascorbinsäure und ihre kosmetische Verwendung
Combination of monosaccharides and ascorbic acid and its use in cosmetic

(30) Priorité: 30.12.2008 FR 0859152; 16.01.2009 US 145119 P
(43) Date de publication de la demande: 07.07.2010
(73) Titulaire: L'Oréal, 75008 Paris (FR)
(72) Inventeur: Laboureau, Julien, 92130, Issy Les Moulineaux (FR); Simonnet, Jean-Thierry, 94230, Cachan (FR); Portes, Pascal, 94130, Nogent sur Marne (FR)
(74) Mandataire: Tezier Herman, Béatrice

(56) Documents cités:
- EP-A- 1 955 692
- WO-A-2004/043469
- WO-A-2004/060393
- WO-A-2008/003900
- FR-A- 2 767 694
- FR-A- 2 768 623
- FR-A- 2 813 789
- FR-A- 2 876 283
- FR-A1- 2 853 539
- FR-A1- 2 900 574
- FR-A1- 2 912 313
- DATABASE GNPD [Online] MINTEL; "Bioderma Bioactive cream", Database accession no. 882653

## Description

La présente invention concerne l'utilisation d' une composition, notamment cosmétique et/ou dermatologique, comprenant dans un milieu physiologiquement acceptable, l'association d'au moins un monosaccharide qu'est le rhamnose et d'au moins un composé additionnel choisi parmi l'acide ascorbique, un de ses analogues et leur mélange.

La peau humaine est constituée principalement de deux couches principales que sont le derme et l'épiderme qui recouvre le derme de manière superficielle. Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes.

La cohésion entre l'épiderme et le derme est assurée par la jonction dermo-épidermique. C'est une région complexe d'une épaisseur de 100 nm environ qui comprend le pôle basal des kératinocytes basaux, la membrane épidermique et la zone sous-basale du derme superficiel. D'un point de vue structurel, des hémidesmosomes, dans lesquels s'insèrent des filaments de kératine (complexe hémidesmosome-tonofilaments), sont répartis sur la membrane plasmique des kératinocytes basaux. Au regard de ces complexes hémidesmosome-tonofilaments, on trouve des filaments d'ancrage qui traversent la membrane basale épidermique. Les filaments d'ancrage sont reliés à la laminine 5 côté épidermique. Enfin, des fibrilles d'ancrage constituent le réseau sous basal. Ce sont des structures curvilinéaires qui prennent naissance et se terminent sur la face profonde de la membrane basale et dans lesquelles viennent s'engager des fibres de collagène I, III et V.

Il a été montré que ces fibrilles d'ancrage, parfaitement visualisables en microscopie électronique, sont composées de collagène de type VII (ci-après collagène VII). Le collagène VII est synthétisé par les kératinocytes et les fibroblastes mais de façon plus importante par les kératinocytes (Aumailley M, Rousselle P. laminins of the dermoepidermal junction. Matrix Biology. 1999, 18: 19-28; Nievers M, Schaapveld R, Sonnenberg A. Biology and function of hemidesmosomes. Matrix Biology, 1999, 18 : 5-17).

Les collagènes sont les protéines majoritaires des matrices extracellulaires de la peau. A ce jour, 20 types de collagènes sont identifiés et notés de I à XX. Les collagènes majoritairement présents dans l'ensemble du derme sont les collagènes de type I et III qui forment la matrice extracellulaire de l'ensemble du derme (ce sont ces collagènes qui constituent 70-80 % du poids sec du derme). Par ailleurs, les collagènes ne sont pas tous synthétisés par les mêmes types cellulaires, les collagènes de type I et III sont essentiellement produits par les fibroblastes dermiques alors que le collagène de type VII est produit par deux catégories de cellules, les kératinocytes et les fibroblastes. La régulation de leur expression diffère d'un collagène à un autre, par exemple, les collagènes I et VII ne sont pas régulés de la même manière par certaines cytokines, en effet, le TNF alpha et la leukoréguline stimulent le collagène VII et régulent négativement le collagène I. Parmi les autres types de collagènes impliqués notamment dans le vieillissement, on peut citer les collagènes XII et VI. Le collagène XII lie les fibrilles de collagène I aux autres composés matriciels au sein du derme papillaire, et régule ainsi les propriétés biomécaniques du tissu cutané : déformabilité et contraction des fibres de collagène en favorisant les glissements de fibres les unes par rapport aux autres. Le collagène VI facilite, comme les protéoglycanes, l'arrangement tridimensionnel des fibres de collagène. La particularité du collagène VI est sa multiplicité d'actions. Il se lie en effet avec un grand nombre de cellules via des récepteurs de type intégrines, et avec de multiples molécules matricielles (collagène IV, fibronectine, biglycan, MAGP-1). Enfin, toutes les molécules de collagène sont des variantes d'un précurseur commun qui est la chaîne alpha du procollagène.

La jonction dermo-épidermique est une structure qui conditionne l'état de surface de la peau. Ainsi, une jonction dermo-épidermique présentant des structures d'ancrage intègres est maintenue plissée, permettant ainsi d'augmenter la surface de la zone de contact entre le derme et l'épiderme, de favoriser les échanges de facteurs diffusibles notamment entre ces deux tissus, de renforcer leur cohésion et d'améliorer l'apparence de l'épiderme. Dans des cas où les structures d'ancrage sont altérées, en particulier du fait d'une déficience de la synthèse de collagène VII, de la ténascine et/ou du fait du vieillissement, ceci provoque un aplanissement de la jonction dermo-épidermique. Les échanges sont amoindris, les deux tissus sont moins solidaires, l'épiderme se plisse et, la peau étant moins ferme et moins tendue, les rides apparaissent et la fragilité de la peau aux agressions mécaniques est accrue.

La ténascine est un constituant majeur de la jonction dermo-épidermique. C'est une glycoprotéine de la matrice extracellulaire, aussi appelée ténascine-C. Sa fonction essentielle réside dans les interactions épithélium-mésenchyme, notamment au cours de l'embryogénèse. Dans la peau, on trouve de la ténascine au niveau sub-épidermique dans le derme papillaire, mais aussi autour des vaisseaux, et des annexes. Son expression est fortement augmentée dans les situations d'hyperprolifération comme le psoriasis, les tumeurs mais aussi dans la cicatrisation. La ténascine est produite dans la peau par les deux types cellulaires majeurs, le kératinocyte et le fibroblaste. Une de ses fonctions réside dans l'adhésion cellulaire. En effet, la forte régulation positive de la ténascine dans les kératinocytes en migration durant la cicatrisation laisse fortement supposer un rôle essentiel d'adhésion des kératinocytes sur le tissu conjonctif, assurant une bonne cohésion dermo-épidermique (Crossin KL. Tenascin: a multifunctional extracellular matrix protein with a restricted distribution in development and disease. J. Cell. Biochem. 1996, 61 : 592-598; Latijnhouwers M, Bergers M, Ponec M, Dijkman H, Andriessen M, Schlkwijk J. Human epidermal keratinocytes are a source of tenascin-C during wound healing. J. Invest Dermatol., 1997, 108 : 776-783 ; Steijlen PM, Maessen E, Kresse H, Van Vlijmen IMJJ, Verstraeten AA, Traupe H, Schalkwijk J. Expression of tenascin, biglycan and decorin in disorders of keratinization. Br. J. Dermatol., 1994, 130 : 564-568).

Avec le vieillissement, le collagène s'amincit et se désorganise, le renouvellement des cellules de la peau est diminué, des rides apparaissent à la surface de la peau, la peau est moins ferme, plus terne. Le vieillissement cutané est conditionné par des caractéristiques génétiques. Par ailleurs, certains facteurs d'environnement comme le tabagisme et surtout l'exposition au rayonnement du soleil l'accélèrent. La peau a ainsi un aspect beaucoup plus âgé sur les zones exposées au soleil comme le dos des mains ou le visage. Ainsi, ces autres facteurs ont également un impact négatif sur le collagène naturel de la peau.

En conséquence, compte tenu du rôle important du collagène au niveau de l'intégrité de la peau et de sa résistance aux agressions externes de type mécaniques, la stimulation de synthèse de ces collagènes, et en particulier du procollagène I et des collagènes VI, VII et XII, apparaît comme un moyen efficace pour pallier les signes du vieillissement cutané.

Pour pallier les inconvénients précités, pour améliorer l'apparence de la peau, pour améliorer ses propriétés mécaniques et éviter les pathologies associées à la carence ou à la déficience cellulaire, du renouvellement cellulaire, ou de certains composés du derme ou de la jonction dermo-épidermique, il semble important de développer des produits qui visent à renforcer ou maintenir le rôle de support et d'élément nourricier que joue le derme, la cohésion entre les différentes couches de la peau, et plus particulièrement la cohésion entre le derme et l'épiderme, en augmentant la prolifération des kératinocytes, en stimulant la prolifération et le métabolisme des fibroblastes et en stimulant la synthèse des collagènes, en particulier le procollagène I, les collagènes VI, VII et XII, et en augmentant la synthèse de la ténascine.

L'épiderme qui recouvre le derme et est en contact direct avec l'environnement extérieur a comme rôle principal de protéger l'organisme de la déshydratation et des agressions extérieures. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac...), appelé « fonction barrière ».

L'épiderme est un épithélium pavimenteux stratifié kératinisé constitué à 90% de kératinocytes. La différenciation progressive des cellules de la membrane basale, qui sépare le derme de l'épiderme, vers la surface de l'épiderme comprend notamment la différenciation des kératinocytes qui migrent vers la surface de la peau où ils desquament.

Le vieillissement de l'épiderme se manifeste principalement par la réduction de son épaisseur. L'atrophie de l'épiderme est la conséquence du ralentissement de la prolifération des kératinocytes et de l'accumulation de kératinocytes sénescents. La couche cornée devient terne.

Les cellules constituant l'épiderme sont délimitées par un domaine lipidique. Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de céramides (sphingolipides).

Ces lipides sont organisés en structures lamellaires spécifiques dont l'intégrité dépend non seulement de la qualité des fractions présentes mais aussi de leur proportion respective. Cette structure lamellaire des lipides du domaine lipidique de l'épiderme est responsable de la fluidité donc de la souplesse de la peau. Les lipides sont également responsables des propriétés « barrière » de l'épiderme, particulièrement du stratum corneum.

Les lipides épidermiques sont synthétisés principalement dans l'épiderme vivant. Ils sont principalement constitués de phospholipides, de sphingolipides, de cholestérol, d'acides gras libres, de triglycérides, d'esters du cholestérol et d'alcanes. Les phospholipides sont essentiels pour la constitution des membranes cellulaires. Ils jouent un rôle important dans la médiation des signaux extracellulaires et la formation de chaînes aliphatiques libres utilisées pour la production d'énergie. Ils constituent un réservoir d'acides gras libres nécessaires à la constitution des sphingolipides. Le cholestérol joue un rôle primordial dans l'hydratation cutanée et dans la fonction "barrière" de l'épiderme. Les acides gras libres jouent un rôle majeur dans le maintien de la structure lamellaire des lipides du stratum corneum, ainsi que dans la constitution des membranes cellulaires où ils sont responsables de la fluidité membranaire mais également de processus physiologiques tels que le fonctionnement de récepteurs ou l'activité enzymatique.

Les céramides, autres lipides jouant un rôle de tout premier ordre dans le métabolisme de l'épiderme, sont nécessaires pour le maintien de la structure multilamellaire des lipides intercornéocytaires. Ils sont également essentiels pour la fonction « barrière » de l'épiderme et pour les échanges en eau, notamment pour pallier les problèmes d'hydratation liés au vieillissement.

Il est connu de la littérature l'utilisation d'agents tels que l'acide ascorbique (vitamine C) ou certains de ses dérivés pour permettre en particulier une augmentation de la synthèse des céramides (J. Invest. Dermatol. 109 :348-355, 1997 ; EP-1 145 706 ; EP-1 145 710). Des tests *in vitro* ont également démontré, par ailleurs, qu'il était possible d'augmenter la synthèse de ténascine et de collagène VII en ajoutant la vitamine C dans le milieu de culture (EP-1 334 714). Par ailleurs, il est connu que l'acide ascorbique, ou l'un de ses dérivés ou analogues, augmente la synthèse du procollagène 1 dans les fibroblastes dermiques (WO2008/003900). Devant la demande sans cesse croissante des utilisateurs de disposer de solutions améliorées pour lutter contre les signes du vieillissement biologique ou actinique de la peau, il existe un besoin de développer des méthodes de soin plus performantes.

Dans ce cadre, la demanderesse a démontré, de manière surprenante, que l'association d'au moins un monosaccharide spécifique et de l'acide ascorbique ou un de ses analogues permettait d'obtenir une action synergique à la fois sur la lipogénèse de l'épiderme, et plus particulièrement sur la synthèse des céramides épidermiques, mais aussi sur la synthèse de ténascine, de collagène VI, XII et/ou VII et/ou sur la stimulation de la synthèse du procollagène I et sur l'activation de la prolifération des kératinocytes et/ou des fibroblastes. Ainsi cette association permet de lutter efficacement contre les signes du vieillissement cutané, au niveau du derme, de la jonction dermo-épidermique et de l'épiderme. En particulier, il est possible de contrer l'atrophie épidermique et/ou dermique liée au vieillissement et/ou de renforcer la cohésion entre le derme et l'épiderme.

La demanderesse a en effet démontré l'activation de la prolifération des kératinocytes et des fibroblastes et la stimulation de la synthèse du procollagène I par le rhamnose. L'utilisation de compositions le contenant permet ainsi de contrer les signes du vieillissement cutané, et en particulier l'atrophie épidermique et/ou dermique liée au vieillissement.

L'utilisation des monosaccharides tels que le rhamnose était restée jusqu'alors inconnue pour les effets biologiques directs exposés plus haut. La demande WO 2007/128939 mentionne toutefois une activité anti-âge obtenue par un effet biomécanique d'un agent tenseur en association avec des composés saccharidiques, qui permettent d'augmenter l'expression des mécanorécepteurs de cellules de la peau. Cette augmentation de l'expression des mécanorécepteurs est décrite comme augmentant la sensibilisation des cellules de la peau à répondre aux effets des tenseurs.

La demande WO 2005/063194 décrit une base galénique à très haute tolérance comprenant notamment du mannose ou du rhamnose. Il est spécifié qu'une telle base galénique ne peut fonctionner qu'en association avec un actif dont elle est seulement le véhicule. Les bases galéniques dermiques et/ou cosmétiques divulguées reposent essentiellement sur la présence des deux polyols que sont le mannitol et le xylitol.

La présente invention concerne donc l'utilisation cosmétique d'une composition comprenant, dans un milieu physiologiquement acceptable, l'association d'au moins un monosaccharide qu'est le rhamnose et leur mélange, et d'au moins un composé additionnel choisi parmi l'acide ascorbique, un de ses analogues et leur mélange, dans laquelle l'analogue de l'acide ascorbique est choisi parmi un sel de l'acide ascorbique tel que l'ascorbate de sodium, l'ascorbylphosphate de magnésium ou de sodium, l'ester acétique de l'acide ascorbique, un ester d'ose de l'acide ascorbique, un sel métallique d'acide ascorbique phosphorylé et leur mélange, et dans laquelle la quantité dudit monosaccharide est comprise entre 0,4 % et 30 % en poids par rapport au poids total de la composition, pour diminuer et/ou prévenir les signes du vieillissement de la peau ou de ses phanères étant l'amélioration de la densité de la peau, sa fermeté et/ou la cohésion de ses différents compartiments, en particulier la cohésion du derme avec l'épiderme. On pourra utiliser l'acide ascorbique, qui est généralement sous forme L car il est le plus souvent extrait de produits naturels, ou ses analogues.

En raison de sa structure chimique (alpha-cétolactone) qui le rend très sensible à certains paramètres de l'environnement tels que la lumière, la chaleur et les milieux aqueux, il pourra être avantageux d'utiliser l'acide ascorbique sous la forme d'un ester d'ose de l'acide ascorbique ou d'un sel métallique d'acide ascorbique phosphorylé.

Les esters d'ose de l'acide ascorbique utilisables dans l'invention sont notamment les dérivés glycosylé, mannosylé, fructosylé, fucosylé, galactosylé, N-acétylglucosaminé, Nacétylmuramique de l'acide ascorbique et leurs mélanges et plus spécialement l'ascorbyl-2 glucoside ou 2-O-α-D glucopyranosyl de l'acide L-ascorbique ou encore le 6-O-D galactopyranosyl de l'acide L-ascorbique. Ces derniers composés, ainsi que leurs procédés de préparation, sont en particulier décrits dans les documents EP-A-487404, EP-A-425066 et J05213736.

De son côté, le sel métallique d'acide ascorbique phosphorylé peut être choisi parmi les ascorbyl phosphates de métal alcalin, les ascorbyl phosphates de métal alcalino-terreux et les ascorbyl phosphates de métal de transition.

Les analogues de l'acide ascorbique sont, plus particulièrement, ses sels, tels que notamment l'ascorbate de sodium, l'ascorbylphosphate de magnésium ou de sodium, l'ester acétique de l'acide ascorbique, ou ses sucres, tels que notamment l'acide ascorbique glycosylé.
Si nécessaire, la stabilisation de l'acide ascorbique vis-à-vis de l'oxydation peut être obtenue par son association avec des dérivés d'anhydride maléique, tel que décrit dans la demande de brevet EP 1374852, ou avec des polymères d'imidazole, tel que décrit dans la demande de brevet FR2832630.

Selon un mode de réalisation préféré de l'invention, le composé additionnel présent dans la composition selon l'invention n'est pas le palmitate d'ascorbyle.

Selon un mode particulier de réalisation de l'invention, la composition selon l'invention ne comprend pas l'association de xylitol et de mannitol. Le rhamnose (ou 6 déoxy mannose) constitue formellement le produit de désoxygénation du mannose en C6. Le rhamnose selon l'invention est sous la forme D ou L ou leur mélange, chaque forme pouvant elle-même être l'anomère alpha et/ou béta. La forme préférée selon l'invention est le L-rhamnose.

Le rhamnose est trouvé dans la nature sous forme L. Le L-rhamnose, est commercialisé par exemple, par la société Danisco Sweeteners® ainsi que la société Symrise. Dans la présente invention, le monosaccharide est plus spécifiquement sous forme de monomère.

La présente invention porte aussi sur l'utilisation, notamment cosmétique ou dermatologique, d'une composition selon l'invention telle que définie précédemment, administrée par voie orale, topique ou par injection cutanée, notamment pour le soin de la peau et/ou du cuir chevelu.

Une composition conforme à l'invention telle que définie précédemment peut notamment être une composition cosmétique pour soin capillaire, en particulier pour la stimulation de la pousse du cheveu, la lutte contre la chute des cheveux, le ralentissement de sa chute ou le renforcement de l'éclat du cheveu.

La présente invention a également pour objet une méthode de traitement, en particulier cosmétique ou thérapeutique, pour diminuer ou prévenir les signes du vieillissement de la peau ou de ses phanères (cheveux, cils, ongles...), par administration à un sujet, de préférence un être humain, d'une quantité efficace d'au moins un monosaccharide tel que défini précédemment en association avec une quantité efficace d'au moins un des analogues de l'acide ascorbique et/ou de l'acide ascorbique lui-même.

La composition ou l'association selon l'invention est plus particulièrement destinée à être appliquée sur les zones du corps ou du visage de personnes présentant des signes de vieillissement de la peau, comme par exemple des rides.

La présente invention concerne également l'utilisation, notamment cosmétique ou dermatologique, de la composition ou de l'association selon l'invention pour diminuer et/ou prévenir les signes du vieillissement de la peau ou de ses phanères.

Selon un mode particulier, la composition utilisée dans la cadre de la présente invention ne comprend pas l'association de xylitol et de mannitol.

La composition ou l'association selon l'invention permet en particulier de stimuler la régénération des cellules de l'épiderme et du derme, au niveau de la peau ou des phanères, en particulier les kératinocytes et les fibroblastes, notamment par augmentation de leur prolifération. On dispose de la sorte d'une méthode, notamment cosmétique, efficace notamment pour lutter contre les signes du chronovieillissement et/ou du photovieillissement.

Les signes du photovieillissement correspondent aux dégradations internes de la peau consécutives à une exposition aux rayonnements ultra-violets (vieillissement actinique). Les signes du chronovieillissement correspondent aux dégradations internes de la peau dues au vieillissement intrinsèque des individus.

Selon une forme de réalisation préférée, l'utilisation selon la présente invention est destinée à améliorer l'éclat du teint, à diminuer et/ou prévenir les caractéristiques des rides et/ou ridules, à améliorer et/ou diminuer le micro-relief de la peau, et/ou à améliorer les propriétés mécaniques de la peau et/ou augmenter la résistance de la peau aux agressions mécaniques, telles que frottements, tensions, frictions et/ou favoriser la réparation de la peau.

Selon un autre aspect de l'invention, l'utilisation de la composition ou de l'association selon l'invention permet d'améliorer la densité de la peau, sa fermeté et/ou la cohésion de ses différents compartiments, en particulier la cohésion du derme avec l'épiderme.

La présente invention concerne également l'utilisation de la composition ou de l'association selon l'invention pour traiter, de manière préventive ou curative, les rides et/ou ridules, la peau flétrie, le manque d'élasticité et/ou de tonus de la peau, l'amincissement du derme, la dégradation des fibres de collagène, la peau molle, la peau amincie, et/ou les dégradations internes de la peau consécutives à une exposition aux rayonnements ultra-violets.

La présente invention concerne également l'utilisation de la composition ou de l'association selon l'invention pour stimuler la régénération de la peau, en particulier de l'épiderme et/ou du derme, grâce à un meilleur renouvellement cellulaire de la peau, en particulier de l'épiderme et/ou du derme.

En agissant au niveau de la jonction dermo-épidermique, en maintenant celle-ci plissée, en permettant ainsi d'augmenter la surface de la zone de contact entre le derme et l'épiderme, de favoriser les échanges entre ces deux tissus, de renforcer leur cohésion et d'améliorer l'apparence de l'épiderme, les compositions selon l'invention permettent d'atténuer les rides et/ou de raffermir la peau.

La composition ou l'association selon la présente invention a pour effet d'améliorer le profil lipidique des épidermes en modifiant la lipogénèse et de renforcer l'intégrité des lipides de la peau, et ainsi d'améliorer la fonction barrière de la peau et/ou la souplesse de la peau.

Un objet particulièrement préféré de la présente invention est l'utilisation de la composition ou l'association selon l'invention pour améliorer le profil lipidique des épidermes en modifiant la lipogenèse, et provoquer en particulier une augmentation de la synthèse des céramides.

Un autre objet préféré de la présente invention est l'utilisation de la composition ou de l'association selon l'invention pour augmenter la prolifération des kératinocytes et/ou des fibroblastes, ou pour stimuler la synthèse de collagènes, en particulier la synthèse de procollagène de type I, de collagène VII, de collagène VI et/ou XII.

Un autre objet particulièrement préféré de la présente invention est l'utilisation de la composition ou de l'association selon l'invention pour augmenter la synthèse de ténascine et/ou de collagène VII.

La quantité des ingrédients actifs, choisis parmi les monosaccharides et l'acide ascorbique et analogues tels que précédemment définis, à mettre en oeuvre selon l'invention dépend de l'effet cosmétique ou thérapeutique recherché, et peut donc varier dans une large mesure. L'homme de l'art peut aisément, sur la base de ses connaissances générales, déterminer les quantités appropriées.

Ainsi, et selon une forme de réalisation préférée, la composition selon l'invention comprend au moins un monosaccharide tel que défini ci-dessus en une quantité comprise entre 0,001 % et 30 % en poids par rapport au poids total de la composition, et en particulier entre 0,1% et 10 % en poids, et plus particulièrement entre 0,5 % et 6 % en poids par rapport au poids total de la composition. Selon une forme de réalisation, et en particulier lorsque le composé additionnel est l'acide ascorbique, la composition selon l'invention comprend au moins un monosaccharide tel que défini ci-dessus en une quantité comprise entre 0,4 % et 30 % en poids par rapport au poids total de la composition, et en particulier entre 0,4% et 10 % en poids, et plus particulièrement entre 0,4 % et 6 % en poids par rapport au poids total de la composition.

Selon une forme de réalisation préférée, la composition selon l'invention comprend la vitamine C et/ou au moins un de ses analogues en une quantité comprise entre 0,001 % et 30 % en poids par rapport au poids total de la composition, et en particulier entre 0,1% et 10 % en poids, et plus particulièrement entre 0,5 % et 6 % en poids par rapport au poids total de la composition.

La composition selon l'invention est adaptée à une administration topique sur la peau ou ses phanères, une administration orale ou une injection cutanée, en particulier sous la forme d'une solution stérile.

De préférence, les administrations topiques selon l'invention se présentent sous forme d'une crème, d'un gel, d'une lotion, d'un lait, d'une huile, d'un onguent, d'une cire, d'une mousse, d'une pâte, d'un sérum, d'une pommade ou d'un shampooing.

De manière également préférée, les administrations orales selon l'invention se présentent sous la forme d'une gélule, d'un comprimé, ou de pilules.

Le monosaccharide selon l'invention et l'acide ascorbique ou un de ses analogues sont plus particulièrement présents dans la composition selon l'invention à titre d'agents (ou ingrédients) actifs, en particulier de seuls agents actifs.

Par « agent actif » ou « ingrédient actif », on entend plus spécifiquement selon l'invention, un composé qui, lorsqu'il est administré à un sujet, en particulier un sujet humain, joue un rôle biologique direct sur l'organisme, en particulier sur la peau ou ses phanères, en particulier sans améliorer l'effet biologique ou mécanique d'un autre composé présent dans la composition selon l'invention.

D'une manière générale, le milieu dans lequel sont compris les principes actifs de la composition telle que définie précédemment est un milieu physiologiquement acceptable, en particulier un milieu cosmétiquement ou pharmaceutiquement acceptable, et peut être anhydre ou aqueux. Il peut ainsi comprendre au moins une phase aqueuse et/ou au moins une phase grasse.

Le milieu physiologiquement acceptable dans lequel les composés selon l'invention peuvent être employés, ainsi que ses constituants, leur quantité, la forme galénique de la composition, son mode de préparation, et son mode d'administration peuvent être choisis par l'homme du métier sur la base de ses connaissances générales en fonction du type de composition recherchée.

Lorsque la composition est une composition destinée à une administration topique, elle peut se présenter avantageusement sous la forme de solutions aqueuses, hydroalcooliques, d'émulsions huile-dans-eau (H/E) ou eau-dans-huile (E/H) ou multiple (triple : E/H/E ou H/E/H), de nanoémulsions, en particulier des nanoémulsions H/E, dont la tailles des gouttes est inférieure à 100 nm, de gels aqueux, ou de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes (tels que décrits dans les demandes FR2709666 et FR2725369)).

Ces compositions sont préparées selon les méthodes usuelles.

En outre, les compositions utilisables selon l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Pour une application locale sur les cheveux ou le cuir chevelu, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Lorsque la composition est une émulsion, la proportion de la phase grasse peut varier d'environ 5 % à 80 % en poids, et de préférence d'environ 2 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

La phase huileuse peut comprendre aussi tout additif habituel liposoluble ou lipodispersible comme indiqué ci-après.

Elle peut notamment comprendre des corps gras tels que des cires, des composés pâteux, des alcools gras, des acides gras. La phase huileuse contient au moins une huile, plus particulièrement au moins une huile cosmétique. On entend par « huile » un corps gras liquide à la température ambiante (25°C).

Comme huiles utilisables dans la composition de l'invention, on peut citer par exemple :
- les huiles hydrocarbonées d'origine animale, telles que le perhydrosqualène;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité, le caprylyl glycol ;

- les esters et les éthers de synthèse, notamment d'acides gras, comme les huiles de formules R1 COOR2 et R1OR2 dans laquelle R1 représente le reste d'un acide gras ou d'unalcool gras comportant de 8 à 29 atomes de carbone, et R2 représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle, l'isopropyl lauroyl sarcosinate, notamment vendu sous le nom commercial Eldew SL 205 de la société Ajinomoto ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam, le mélange de n-undécane (C11) et de n-tridécane (C13) commercialisé sous la référence de CETIOL UT par la Société Cognis ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; lespolydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; ou
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » et « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination « Dow Corning 5200 Formulation Aid » par la société Dow Corning et le Cetyl dimethicone copolyol vendu sous la dénomination « Abil EM 90® » par la société Goldschmidt. On peut aussi utiliser comme tensioactif d'émulsions E/H, un
organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

Ces compositions peuvent être également des émulsions H/E stabilisées par des particules comme par exemple des particules polymériques décrites dans le brevet FR2760641, des polymères amphiphiles réticulés ou non tels que décrits dans les demandes : FR2853543 et FR2819175.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs notamment l'éthanol, l'isopropanol, le dipropylène glycol, le butylène glycol et le propylène glycol.

Comme gélifiants hydrophiles utilisables dans l'invention, on peut citer à titre d'exemples non limitatifs, les polymères carboxyvinyliques (carbomer®), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Lorsque le monosaccharide est administré par voie orale, la composition le contenant peut être avantageusement sous la forme d'une gélule, d'un comprimé, ou de pilules. Lorsque le monosaccharide est administré par injection cutanée, la composition le contenant peut être en particulier sous la forme d'une solution stérile.

Les compositions de l'invention peuvent contenir d'autres actifs hydrophiles ou lipophiles. Ces actifs sont notamment choisis parmi les agents anti-oxydants, les agents dermo-relaxants ou dermodécontractants, les agents anti-âge, les agents anti-glycation, les agents stimulants la synthèse de macromolécules dermiques ou épidermiques et/ou empêchant leur dégradation, les agents stimulants la prolifération des fibroblastes ou des kératinocytes et/ou la différenciation des kératinocytes, les agents favorisant la maturation de l'enveloppe cornée, les inhibiteurs de NO synthase et les agents stimulant le métabolisme énergétique des cellules. Des listes de ces actifs sont données à la suite à titre illustratif, et ne doivent en aucune façon être considérées comme limitatives.

### Agents anti-âge :

Parmi les actifs connus pour lutter contre les signes du vieillissement, notamment cutané, on peut citer notamment :
la vitamine B3, le coenzyme Q10 (ou ubiquinone), la vitamine B9, la vitamine E, les dérivés de la vitamine E tels que le dérivé phosphaté comme par exemple le TPNA® commercialisé par la société Showa Denko, le resvératrol ou ses dérivés, comme par exemple le resveratrate® commercialisé par la société Estée Lauder, le rétinol ou ses dérivés, et leur mélange.

### Agents anti-glycation:

Par « agent anti-glycation », on entend un composé prévenant et/ou diminuant la glycation des protéines de la peau, en particulier des protéines du derme, telles que le collagène.

Comme agents anti-glycation, on peut citer notamment les extraits végétaux de la famille des *Ericaceae,* tels qu'un extrait de myrtille (*Vaccinium angusfifollium, Vaccinium myrtillus*), par exemple celui vendu sous la dénomination « BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802 420, respectivement), les dihydroxystilbènes et leurs dérivés, les polypeptides d'arginine et de lysine tels que celui vendu sous la dénomination « AMADORINE® » par la société SOLABIA, le chorhydrate de carcinine (commercialisé par Exsymol sous la dénomination « ALISTIN®»), un extrait d'*Hélianthus annuus* comme l'Antiglyskin® de SILAB, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777® de Laboratoires Sériobiologiques, un extrait de thé noir comme le Kombuchka® de Sederma et leurs mélanges.

Comme agents anti-glycation préférés, on citera les extraits de myrtille (*Vaccinium myrtillus*) et l'extrait de thé noir.

### Agents stimulant la synthèse de macromolécules dermiques et/ou épidermiques et/ou empêchant leur dégradation :

Parmi les actifs stimulant les macromolécules du derme ou empêchant leur dégradation, on peut citer ceux qui agissent :
- soit sur la synthèse du collagène, tels que les extraits de Centella asiatica, les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; les hormones végétales telles que les auxines et les lignanes ; l'acide folique ; et un extrait de Medicago sativa (alfafa) tel que celui commercialisé par SILAB sous la dénomination Vitanol®; un extrait peptidique de noisette tel que celui commercialisé par la société Solabia sous la dénomination Nuteline C®; et l'arginine.
- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol® de Silab, un extrait d'aphanizomenon flos-aquae (cyanophycée) commercialisée sous la dénomination Lanablue® par Atrium Biotechnologies, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon; un extrait de litchi ; la DIPALMITOYL HYDROXYPROLINE commercialisé par Seppic sous le nom SEPILIFT DPHP® : Baccharis genistelloide ou Baccharine commercialisé par SILAB, un extrait de moringa tel que l'Arganyl LS 9781® de Cognis ; l'extrait de sauge décrit dans la demande FR-A-2812544 de la famille des labiées (*salvia officinalis* de la société Flacksmann), l'extrait de Rhododendron, le blueberry extract, un extrait de *vaccinium myrtillus* tel que ceux décrits dans la demande FR-A-2814950.
- soit sur la synthèse de molécules appartenant à la famille des élastines (élastine et fibrilline), tels que : le rétinol et dérivés en particulier le palmitate de rétinol ; l'extrait de *Saccharomyces Cerivisiae* commercialisé par la société LSN sous la dénomination commerciale Cytovitin® ; et l'extrait d'algue *Macrocystis pyrifera* commercialisé par la société SECMA sous la dénomination commerciale Kelpadelie®; un extrait peptidique de noisette tel que celui commercialisé par la société Solabia sous la dénomination Nuteline C®.
- soit sur l'inhibition de la dégradation de l'élastine tels que l'extrait peptidique de graines de *Pisum sativum* commercialisé par la société LSN sous la dénomination commerciale Parelastyl® ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en C₁-C_{6.} ; un extrait de peptides de riz tel que la Colhibin® de Pentapharm, ou un extrait de Phyllanthus emblica tel que l'Emblica® de Rona.
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que la Laminaïne® de Secma ; l'essence de Mamaku de Lucas Meyer, un extrait de cresson (Odraline® de Silab).
- soit sur la synthèse de la fibronectine, tels que l'extrait de zooplancton Salina commercialisé par la société SEPORGA sous la dénomination commerciale GP4G® ;
l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC; et l'extrait de zooplancton Salina commercialisé par la société SEPORGA sous la dénomination commerciale GP4G®; Tripeptide de Cuivre de PROCYTE ; un extrait peptidique de *Voandzeia substerranea* tel que celui commercialisé par la société Laboratoires Sérobiologiques sous la dénomination commerciale Filladyn LS 9397®.

De préférence, on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de *Saccharomyces cerevisiae,* un extrait de *Laminaria ochroleuca,* l'essence de Mamaku, un extrait de cresson et leurs mélanges.

Comme actifs préférés stimulant la synthèse de macromolécules dermiques et/ou épidermiques et/ou empêchant leur dégradation, on peut citer :
les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; l'acide folique ; un extrait de Medicago sativa (alfafa) tel que celui commercialisé par SILAB sous la dénomination Vitanol® ; un extrait peptidique de noisette tel que celui commercialisé par la société Solabia sous la dénomination Nuteline C® ; l'arginine ; un extrait d'aphanizomenon flos-aquae (cyanophycée) commercialisée sous la dénomination Lanablue® par Atrium Biotechnologies, l'extrait de malt commercialisé par la société COLETICA sous la dénomination commerciale Collalift® le lycopène ; un extrait de litchi ; un extrait de moringa tel que l'Arganyl LS 9781® de Cognis ; un extrait de *vaccinium myrtillus* tel que ceux décrits dans la demande FR-A-2814950 ; le retinol et dérivés en particulier le palmitate de rétinol ; l'extrait de *Saccharomyces Cerivisiae* commercialisé par la société LSN sous la dénomination commerciale Cytovitin® ; un extrait peptidique de noisette tel que celui commercialisé par la société Solabia sous la dénomination Nuteline C®; l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valylglycine ou acetyl trifluoromethyl phenyl valylglycine, ou un ester de celui-ci avec un alcool en C₁-C_{6.} ; un extrait de peptides de riz tel que la Colhibin® de Pentapharm, ou un extrait de Phyllanthus emblica tel que l'Emblica® de Rona ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que la Laminaïne® de Secma ; l'essence de Mamaku de Lucas Meyer, l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC.

### Agents stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; et les hormones végétales telles que les giberrellines et les cytokinines ; un extrait peptidique de noisette tel que celui commercialisé par la société Solabia sous la dénomination Nuteline C®.

De préférence on utilisera un agent favorisant la prolifération et/ou la différenciation des kératinocytes.

Les agents stimulant la prolifération des kératinocytes, utilisables dans la composition selon l'invention, comprennent notamment : le phloroglucinol, l'extrait de feuille d'hydrangea macrophylla comme l'Amacha liquid E® d'Ichimaru Pharcos, un extrait de levure tel que le Stimoderm® de CLR ; l'extrait de *Larrea divaricata* tel que le Capislow® de Sederma, les mélanges d'extraits de papaye, de feuilles d'olivier et de citron tel que la Xyléine® de Vincience, l'extrait de feuille d'hydrangea macrophylla comme l'Amacha liquid E® d'Ichimaru Pharcos, le rétinol et ses esters dont le palmitate de rétinyle, les extraits de tourteaux de noix commercialisés par Gattefosse et les extraits de *solanum tuberosum* tel que le Dermolectine® commercialisé par Sederma.

Parmi les agents stimulant la différenciation des kératinocytes comprennent par exemple les minéraux tels que le calcium; un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; et les lignanes tels que le sécoisolaricirésinol, le rétinol et ses esters dont le palmitate de rétinyl.

Comme agents stimulant la prolifération et/ou la différenciation des kératinocytes, on peut citer encore les oestrogènes tels que l'oestradiol et homologues ou les cytokines.

Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; un extrait peptidique de noisette tel que celui commercialisé par la société Solabia sous la dénomination Nuteline C®; le phloroglucinol, un extrait de levure tel que le Stimoderm® de CLR ; un extrait peptidique de lupin tel que celui commercialisé par la société SILAB sous la dénomination commerciale Structurine®; un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; le rétinol et ses esters dont le palmitate de rétinyle.

### Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance® de National Starch et les autres actifs mentionnés dans la demande de L'OREAL FR2877220.

### Inhibiteurs de NO-synthases

L'agent ayant une action d'inhibiteur de NO synthase peut être choisi parmi les OPC (oligomères procyanidoliques) ; les extraits de végétal de l'espèce *Vitis vinifera* notamment commercialisés par la société Euromed sous la dénomination Leucocyanidines de raisins extra, ou encore par la société Indena sous la dénomination Leucoselect®, ou enfin par la société Hansen sous la dénomination Extrait de marc de raisin ; les extraits de végétal de l'espèce *Olea europaea* de préférence obtenus à partir de feuilles d'olivier et notamment commercialisés par la société VINYALS sous forme d'extrait sec, ou par la société Biologia & Technologia sous la dénomination commerciale Eurol® BT ; les extraits d'un végétal de l'espèce *Gingko biloba* de préférence un extrait aqueux sec de ce végétal vendu par la société Beaufour sous le nom commercial Ginkgo biloba extrait standard et leurs mélanges.

### Agents stimulant le métabolisme énergétique des cellules

L'actif stimulant le métabolisme énergétique des cellules peut par exemple être choisi parmi la biotine, un extrait de *Saccharomyces cerevisiae* tel que le Phosphovital® de Sederma, le mélange de sels de sodium, de manganèse, de zinc et de magnésium d'acide pyrrolidone carboxylique comme le Physiogenyl® de Solabia, un mélange de gluconate de zinc, de cuivre et de magnésium tel que le Sepitonic M3® de Seppic et leurs mélanges ; un beta-glucan issu de *Saccharomyces cerevisiae,* tel que celui commercialisé par la société Mibelle AG Biochemistry.

L'invention porte également sur un procédé de traitement cosmétique de la peau destiné à diminuer ou prévenir les signes du vieillissement de la peau ou de ses phanères (cheveux, cils, ongles, ...), comprenant au moins une étape consistant à appliquer sur la peau au moins une composition telle que définie précédemment.

Le procédé selon l'invention comprend plus spécifiquement au moins une étape consistant à appliquer sur la peau de personnes présentant une peau présentant au moins l'un des signes de vieillissement cutané rappelés précédemment au moins une composition telle que définie précédemment.

Plus particulièrement, il comprend au moins une étape consistant à appliquer sur la peau de personnes présentant une peau ou une zone de peau vieillie, ridée, ou molle et/ou flasque ou sur des zones du corps présentant une perte d'élasticité et/ou de fermeté et/ou de tonicité au moins une composition telle que définie précédemment.

La composition selon l'invention peut être appliquée sur la partie de la peau ou des phanères à traiter, en particulier sur le visage, le corps, le cou, les mains, les cheveux ou le cuir chevelu, de préférence de façon quotidienne ou pluriquotidienne. L'application pourra être par exemple renouvelée tous les jours pendant une période variable selon les effets souhaités, généralement de 3 à 6 semaines, mais pourra être prolongée ou poursuivie en continu.

Selon une alternative, la composition selon l'invention peut être administrée par voie injectable en association ou non avec des produits de comblement. En effet, l'une des solutions retenues pour lutter contre les rides et/ou la perte de volume des tissus mous est l'utilisation de produits de comblement (ou "filler"). Ce comblement peut être réalisé par l'utilisation de produits non résorbables, tels que des gels de polyacrylamide ou des particules de polyméthylméthacrylate (PMMA). Néanmoins, ces composés peuvent entrainer des réactions d'intolérance du type inflammation ou hypersensibilité.

On privilégie l'utilisation de composants résorbables, tels que les protéines, les graisses, le collagène ou l'acide hyaluronique. Mais ces composés sont dégradés assez rapidement dans l'organisme, ce qui réduit leur efficacité. Pour y remédier il faut donc procéder à une réticulation plus ou moins poussée de ces composants. A ce jour, l'acide hyaluronique utilisé dans des formes pharmaceutiques ou des dispositifs médicaux se présente sous la forme d'un gel de hyaluronate de sodium. Le monosaccharide selon l'invention ou les compositions les contenant pourront également être appliqués par mésothérapie. La mésothérapie est une technique de traitement par injection intraépidermique et/ou intradermique et/ou sous-cutanée de produit(s) actif(s), comme par exemple des micro-nutriments, des vitamines, et/ou de l'acide hyaluronique. Les compositions sont administrées selon cette technique par injection sous forme de multiples gouttelettes de faible taille au niveau de l'épiderme, de la jonction dermo-épidermique et/ou du derme afin, notamment, de réaliser un nappage sous-cutané. La technique de mésothérapie est notamment décrite dans l'ouvrage « Traité de mésothérapie » de Jacques LE COZ, édition Masson, 2004. La mésothérapie faite sur le visage est également appelée mésolift, ou également sous le terme anglosaxon de « mesoglow ».

Ainsi, un autre objet de la présente invention peut être un dispositif, en particulier un dispositif médical, comprenant une quantité efficace d'au moins un monosaccharide tel que défini précédemment en association avec une quantité efficace d'au moins un des analogues de l'acide ascorbique, ou de l'acide ascorbique lui-même. Ce dispositif peut être adapté à une injection intraépidermique et/ou intradermique et/ou sous-cutanée. L'association d'actifs telle que définie ci-dessus est mise en solution dans un milieu stérile. Ledit dispositif peut comprendre au moins un autre composé, comme au moins un produit résorbable ou non, tel que ceux mentionnés ci-dessus, éventuellement réticulé.

Ledit dispositif peut être par exemple une seringue avec une aiguille ou encore un dispositif injecteur sans aiguille, tel que ceux utilisés dans la technique de soin connue sous le nom de mésothérapie. Un kit comprenant un dispositif peut être également envisagé, ledit kit comprenant un dispositif, en particulier une seringue ou un dispositif injecteur, et au moins l'association d'actifs, monosaccharide(s) et acide ascorbique ou analogue(s), tel que défini ci-dessus. Ledit kit peut comprendre également une aiguille. Ledit dispositif peut se trouver prêt à l'emploi, c'est à dire pré-rempli, ou doit être rempli lors de l'utilisation. Dans ce dernier cas, une composition ou un autre dispositif (comme une ampoule) comprend ladite l'association d'actifs, monosaccharide(s) et acide ascorbique ou analogue(s), éventuellement en association avec au moins un autre composé actif, comme au moins un produit résorbable ou non, tel que les produits de comblement mentionnés ci-dessus, éventuellement réticulé.

L'injection de l'association selon l'invention peut être réalisée simultanément à, ou avant ou après, l'application sur la peau ou ses phanères d'une autre composition cosmétique ou pharmaceutique, de préférence dermatologique, comprenant, dans un support physiologiquement acceptable, au moins un autre actif, tel que cité ci-dessus.

Selon un autre aspect, l'invention concerne également un ensemble cosmétique comprenant : i) un récipient délimitant au moins un compartiment, ledit récipient étant fermé par un élément de fermeture ; et ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier. L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur. L'applicateur peut être sous forme d'un pinceau, tel que décrit par exemple dans le brevet FR 2 722 380. Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage.

Alternativement, le couplage entre l'élément de fermeture et le récipient se fait autrement que par vissage, notamment via un mécanisme à baïonnette, par encliquetage, serrage, soudage, collage, ou par attraction magnétique. Par « encliquetage » on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique, comme le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube. Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le contenu des brevets ou demandes de brevets cités précédemment sont incorporés par référence dans la présente demande.

Selon un mode particulier, l'invention concerne un ensemble cosmétique comprenant :
- une composition A contenant au moins un composé choisi parmi l'acide ascorbique, un de ses analogues et leurs mélanges,
- une composition B, conditionnée de manière séparée de la composition A, comprenant au moins un monosaccharide choisi parmi le mannose, le rhamnose et leur mélange.

L'invention concerne enfin un procédé de traitement cosmétique ou dermatologique comprenant au moins une étape d'administration, en particulier d'application topique, sur la peau et/ou ses phanères, de la composition A et au moins une étape d'administration, en particulier d'application topique sur la peau et/ou ses phanères, de la composition B.

L'administration de la composition A selon l'invention peut être réalisée simultanément à, ou avant ou après, l'administration de la composition B. Comme spécifié précédemment, l'administration de la composition A et de la composition B peut être réalisée par voie topique, orale ou par injection.

Selon une alternative, on administre en premier lieu la composition A et en second lieu la composition B. Selon une autre alternative, on administre en premier lieu la composition B et en second lieu la composition A.

Les compositions A et B peuvent être conditionnées séparément à l'intérieur de deux compartiments, formés soit par deux récipients distincts, soit à l'intérieur d'un dispositif unitaire. Par « dispositif unitaire », on entend un dispositif par lequel les deux compartiments sont solidaires l'un de l'autre. Un tel dispositif peut être obtenu par un procédé de moulage en une seule pièce des deux compartiments, notamment en un matériau thermoplastique. Il peut également résulter de toute forme d'assemblage, notamment par collage, soudage, ou autre encliquetage.

Selon un premier mode de réalisation, les deux récipients sont indépendants l'un de l'autre. De tels récipients peuvent se présenter sous diverses formes. Il peut s'agir notamment de tubes, de flacons ou de bidons.

L'un et/ou l'autre des récipients peuvent être surmontés d'une pompe à actionnement manuel surmontée d'un bouton poussoir pour l'actionnement de la pompe et la distribution de la composition via au moins un orifice de distribution.

Alternativement, l'un et/ou l'autre des récipients sont pressurisés, notamment au moyen d'un agent propulseur, en particulier un gaz propulseur. Dans ce cas, le (ou les) récipient(s) est (sont) équipé(s) d'une valve surmontée par un bouton poussoir équipée d'une buse ou de tout autre moyen de diffusion pour la distribution du produit.

Le propulseur peut être en mélange avec la composition à distribuer ou séparé, notamment via un piston apte à coulisser à l'intérieur du récipient, ou via les parois souples d'une poche à l'intérieur de laquelle est disposée la composition.

Les récipients peuvent être constitués de matériaux divers : plastique, verre, ou métal.

Alternativement encore, les deux compartiments sont formés de deux compartiments concentriques formés à l'intérieur d'un tube, et sont surmontés d'une pompe sans reprise d'air équipée d'un bouton poussoir à un ou deux orifices de distribution. A l'intérieur du tube est prévu un piston qui remonte en direction de la pompe au fur et à mesure que les compositions sont prélevées à l'intérieur des récipients. De tels modes de distribution sont utilisés notamment pour la distribution de pâtes dentifrices.

### Légendes des Figures

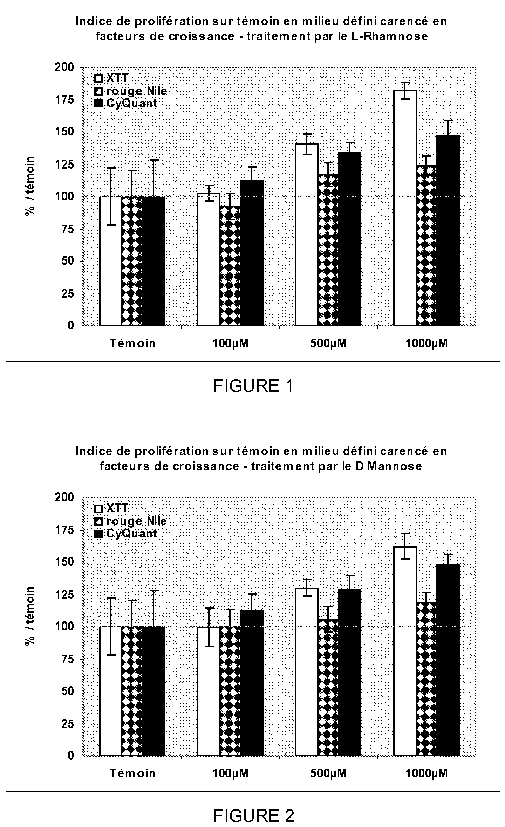

**Figure 1** **:** Diagramme schématisant les résultats obtenus pour la prolifération des kératinocytes, en présence d'un témoin, en présence de différents marqueurs, en milieu carencé en facteurs de croissance, et avec ajout de différentes concentrations en L-Rhamnose reportées en abscisse. Les valeurs reportées en ordonnée correspondent aux pourcentages de cellules marquées mesurés par rapport au témoin.
**Figure 2** **:** Diagramme schématisant les résultats obtenus pour la prolifération des kératinocytes, en présence d'un témoin, en présence de différents marqueurs, en milieu carencé en facteurs de croissance, et avec ajout de différentes concentrations en D-Mannose reportées en abscisse. Les valeurs reportées en ordonnée correspondent aux pourcentages de cellules marquées mesurés par rapport au témoin.

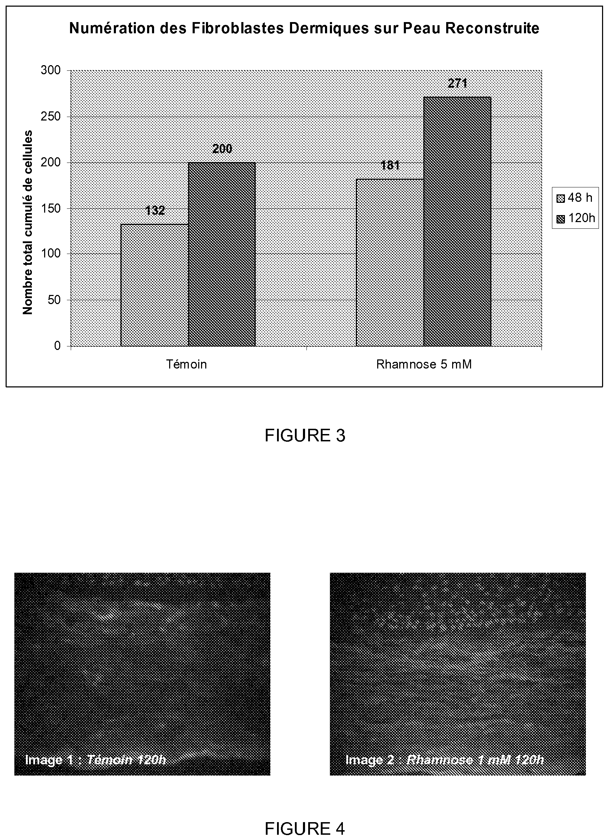

**Figure 3** **:** Diagramme représentant le nombre de fibroblastes mesurés entre une peau reconstruite complète témoin non traité, à gauche, et une peau reconstruite complète traitée par 5 mM de rhamnose, à droite. Les fibroblastes sont comptés à différents stades du traitement. Ainsi, pour chaque type de peau la colonne de gauche correspond à la numérotation effectuée à 48 h et la colonne de droite correspond à la numérotation effectuée à 120 h de traitement.
**Figure 4** **:** Photographies de coupe à congélation de peau reconstruite de 7µM d'épaisseur. Le niveau de fluorescence se matérialise par les tâches blanche du cliché en noir et blanc, il est proportionnel à la quantité de procollagène de type I. A gauche figure la peau témoin et à droite la peau traitée par 1 mM de rhamnose.

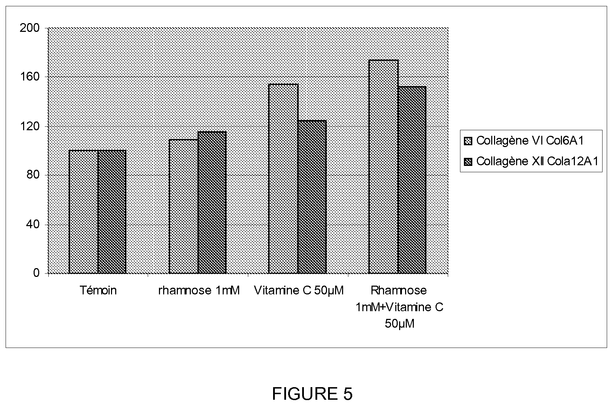

**Figure 5** **:** Diagramme représentant la quantité de collagènes de types VI et XII synthétisés par les fibroblastes du derme en présence ou en absence de rhamnose et/ou de vitamine C.

L'invention est illustrée plus en détail dans les exemples suivants qui sont présentés à titre illustratif et non limitatif du domaine de l'invention.

### Exemples

### Exemple 1 : Prolifération des kératinocytes

### Protocole

Les kératinocytes (lignée HaCat) sont cultivés dans deux conditions : milieu de culture défini complet (condition standard) et milieu de culture carencé en facteurs de croissance. Ce milieu carencé entraîne un retard maitrisé de la prolifération cellulaire. Dans ces conditions, il est alors possible de mesurer les effets de composés, capables de compenser la carence en facteurs de croissance du milieu de culture et donc de relancer la multiplication cellulaire et /ou de stimuler leur métabolisme.

La prolifération kératinocytaire est mesurée au moyen de trois marqueurs sur la même population cellulaire : le taux d'ADN qui est proportionnel au nombre de cellules (sonde Cyquant), le taux de lipides polaires constitutifs de membranes cellulaires (sonde Rouge Nil) et la respiration mitochondriale, qui réflète le métabolisme cellulaire général (sonde XTT).

### Résultats

Les résultats sont donnés aux figures 1 et 2.
Les deux monosaccharides Rhamnose et Mannose démontrent leur capacité à activer la prolifération des kératinocytes, lorsque ceux-ci sont cultivés en milieu appauvri en facteurs de croissance, condition de culture qui retarde significativement leur croissance cellulaire.

Cette activation de la prolifération cellulaire par les deux composés se manifeste par un nombre de cellules plus important par rapport au témoin non traité.
Ce nombre augmenté de cellules se matérialise par un taux d'ADN (Cyquant), un taux de lipides polaires (signal Rouge Nil) et une respiration mitochondriale (signal XTT) significativement augmentés lorsque les monosaccharides sont évalués à 1 mM. A 500 µM, les deux molécules présentent déjà une efficacité.
Les deux monosaccharides mannose et rhamnose exercent donc une influence sur la prolifération des kératinocytes. Ils activent la prolifération des kératinocytes cultivés en milieu appauvri en facteur de croissance, ce qui se manifeste par un nombre de cellules plus important par rapport au témoin non traité.

Le rhamnose et le mannose présentent donc une efficacité anti âge intéressante en venant booster le renouvellement épidermique et lutter contrer l'atrophie épidermique liée au vieillissement.

### Exemple 2 : Prolifération des fibroblastes

### Protocole

Le rhamnose a été étudié sur un modèle de peau reconstruite complète afin de mesurer son efficacité anti âge au niveau du compartiment dermique. Brièvement, le modèle de peau reconstruite utilisé est celui décrit par Bell et al, (Bell E. et al, The reconstitution of living skin, J Invest Dermatol, 1983, Jul ;81) : il comporte un équivalent dermique sur lequel est reconstruit un épiderme pluristratifié ; l'équivalent dermique est fabriqué à partir de collagène acido soluble, de milieu de culture contenant du sérum et de fibroblastes humains normaux adultes. Après 5 jours de rétraction, cet équivalent est ensemencé avec des kératinocytes puis cultivé durant 6 jours en immersion et 7 jours à émersion afin d'obtenir un épiderme pluristratifié et différencié présentant une couche cornée.

La peau reconstruite est traitée par du rhamnose à 5 mM pendant 2 jours et 5 jours dans le milieu de culture ; à l'issue du traitement, les peaux reconstruites sont incluses en Tissue Tek en vue de coupes à congélation de 7µM d'épaisseur au cryostat. Les coupes réalisées sont ensuite marquées à l'iodure de propidium pour marquer l'ADN des noyaux des fibroblastes en vue de leur numération. 3 coupes à congélation sont réalisées aléatoirement sur chaque peau reconstruite ; sur chaque coupe, 2 champs microscopiques (objectif X25) sont analysés en microscopie à fluorescence et photographiés. La numération des fibroblastes dermiques est donc réalisée pour chaque peau reconstruite sur 6 images au total représentant les 6 champs microscopiques considérés. Le nombre de fibroblastes dermiques est comparé entre la peau témoin et celle traitée par le rhamnose aux deux temps de la cinétique.

### Résultats

Les résultats sont donnés à la figure 3.
Il a été constaté que le rhamnose induit la stimulation de la croissance des fibroblastes dermiques de la peau reconstruite dès 48 heures de traitement, stimulation confirmée à 120 h de traitement, avec entre 30 à 35% de cellules en plus (voir figure 3). A noter que cette stimulation s'accompagne d'une stimulation de la synthèse de procollagène 1 à 5 mM, ainsi qu'à 1 mM, ce qui peut également résulter du nombre accru des fibroblastes responsable de la sécrétion de cette protéine majeure de la matrice extracellulaire.

Ces deux effets complètent l'activité anti-âge du rhamnose déjà mesurée sur le compartiment épidermique, en venant stimuler la prolifération et le métabolisme du fibroblaste, cellule majeure du compartiment dermique.

### Exemple 3 : Synthèse de Procollagène 1

Il a été procédé également sur d'autres séries de coupes à congélation à la détection classique par immunofluorescence indirecte du procollagène de type I au niveau du derme de la peau reconstruite (Anticorps anti procoll 1 *(MAB 1912 Millipore)* + conjugué couplé à FITC *(112-095-068 Jackson Immunoresearch)).* Afin de bien se repérer au sein de l'architecture cutanée lors de l'examen microscopique des coupes, les noyaux cellulaires des kératinocytes et des fibroblastes sont localisés grâce à leur marquage à l'iodure de propidium, comme décrit plus haut. 3 coupes à congélation sont réalisées aléatoirement sur chaque peau reconstruite et sur chaque coupe, 2 champs microscopiques (objectif X25) sont analysés en microscopie à fluorescence et photographiés. Les niveaux de fluorescence proportionnels à la quantité de procollagène de type I sont comparés entre la peau témoin et la peau traitée par le rhamnose.

Sur l'image 1, figure 4, correspondant à une coupe de la peau reconstruite témoin à 120h de culture, la présence du procollagène de type 1 synthétisé par les fibroblastes dermiques, se matérialise par la fluorescence verte située dans la partie inférieure de l'image. On devine sur la partie supérieure de l'image, la partie basale de l'épiderme, tissu très cellulaire, visualisable par les nombreux noyaux des kératinocytes. On visualise également dans le derme ; tissu beaucoup moins cellulaire, la distribution aléatoire des fibroblastes au sein de la matrice extracellulaire dermique. Sur l'image 2, figure 4, correspondant par exemple, à une coupe de la peau reconstruite traitée par le Rhamnose à 1mM pendant 120 heures, on constate une nette augmentation de la fluorescence verte comparativement à celle observée pour la peau témoin (image 1) ainsi qu'une distribution du signal fluorescent matérialisant bien l'aspect fibrillaire du procollagène de type I néosynthétisé. Cette augmentation de la fluorescence générale indique que le traitement par le rhamnose a fortement stimulé la synthèse du procollagène de type I par les fibroblastes.

Ces résultats montrent bien la capacité du rhamnose à stimuler le métabolisme du fibroblaste, métabolisme qui au cours du vieillissement, se déséquilibre plus vers la dégradation de la matrice extracellulaire que vers son renouvellement.

Le rhamnose en stimulant à la fois le métabolisme et la croissance des fibroblastes dermiques démontre bien son efficacité anti-âge sur le derme, efficacité complémentaire de celle mesurée vis-à-vis du compartiment épidermique.

### Exemple 4: Association Rhamnose et Acide Ascorbique, mise en évidence de la synergie d'action de la vitamine C et du Rhamnose sur la synthèse des céramides épidermiques.

### Protocole

### Pour l'acide ascorbique seul (sans rhamnose) :

L'acide ascorbique est testé sur un équivalent de peau vendu par la société EPISKIN (Lyon, France), après culture de celui-ci pendant 7 jours. Le mode opératoire et les quantités d'acide ascorbique utilisés sont identiques au protocole détaillé à la suite pour l'association avec le rhamnose.

### Pour l'association d'actifs selon l'invention :

L'association de l'acide ascorbique et du rhamnose est testée sur un équivalent de peau vendu par la société EPISKIN (Lyon, France), après culture de celui-ci pendant 7 jours. Les milieux de culture et d'essais sont ceux inclus dans le kit vendu par le fournisseur. L'acide ascorbique a été testé à 100 et/ou 200 µg/ml en association avec le Rhamnose à 5 mM dans le milieu de culture. Les épidermes reconstruits sont traités durant 24 heures. Le témoin est constitué par un équivalent d'épiderme identique ne subissant aucun traitement. L'épiderme reconstruit est incubé pendant une nuit avec de l'acétate 14C (2 µCi/ml), pour suivre la synthèse de lipides. A la fin de l'incubation, on détache l'équivalent d'épiderme de son support collagènique.

La préparation des lipides de l'équivalent d'épiderme et leur analyse par HPTLC ou chromatographie sur couche mince haute performance sont réalisées selon la technique et avec les tampons décrits par M. Ponec (1991, Adv. Lipid Res. 24 :83-117).

En fin de migration, l'analyse densitométrique de l'autoradiographie est réalisée à l'aide d'un densitomètre de marque FUJI, modèle 1800. On compare les quantités de lipides épidermiques totaux entre les épidermes témoins et ceux traités par l'association. En complément, on peut également analyser plus particulièrement la modulation des classes de lipides spécifiques comme les phospholipides, les acides gras libres, les céramides et cérébrosides, et le cholestérol.

### Résultats

On note une production de lipides épidermiques supérieure en quantité, lorsque les épidermes reconstruits sont traités par l'association de l'acide ascorbique et du rhamnose comparativement à l'acide ascorbique seul.

### Exemple 5 : Association Rhamnose et Acide Ascorbique, mise en évidence de la synergie d'action de la vitamine C et du Rhamnose sur la synthèse des collagènes de types VI et XII par les fibroblastes.

Cet exemple démontre l'effet synergique de l'association de la vitamine C avec du L-rhamnose sur l'expression de la chaîne alpha 1 des collagènes VI et XII de la matrice extra-cellulaire.

### Protocole

Les fibroblastes dermiques (passage 9) humains proviennent de plasties mammaires ou abdominales. Le milieu de culture mis en oeuvre est le DMEM supplémenté en :
- Sérum de veau foetal (SVF), 10 %,
- L-Glutamine, 2 mM (Invitrogen 25030024),
- Penicilline (50 Ul/ml), streptomycine (50 µg/ml), (Invitrogen 15070063),
- Pyruvate de sodium (Invitrogen 11360039), et
- Acides aminés non essentiels (Invitrogen 11140035).

Les fibroblastes sont ensemencés (J0) en milieu DMEM supplémenté tel que décrit ci-dessus, incubés à 37°C sous 5% CO₂ dans une atmosphère saturée en eau. Après adhésion des cellules (J1), le milieu de culture est éliminé puis remplacé par du milieu contenant la vitamine C, le L-Rhamnose ou leur association. Les différentes conditions de stimulation (vitamine C 25 µM, 50 µM, Rhamnose 1 mM et les différentes associations) ont été réalisées en triplicate (boîte 12 puits).

Les études d'expression des différents collagènes ont été réalisées à J3 et à J5.

### Extraction et purification des ARNs :

Pour chacune des conditions et à chaque temps (J3 et J5), les surnageants sont éliminés et les tapis cellulaires lysés. Les ARNs totaux sont ensuite extraits et purifiés. L'ensemble de ces opérations est réalisé par la mise en oeuvre du kit Qiagen n° 79254.

### Quantification et qualification des ARNs :

Les ARNs sont ensuite quantifiés en fluorescence selon le protocole Ribogreen (Quan-it, Ribogreen RNA assay kit, invitrogen R11490), lecture au Tecan Safire. La qualité des ARNs est appréciée par suivi du profil électrophorétique.

Les concentrations finales de vitamine C, de rhamnose et de leurs associations testées sont les suivantes : 50µM de Vitamine C, 1mM de L-Rhamnose, et association : 50 µM Vitamine C + 1 mM de L-Rhamnose.

**Résultats**

| | Collagène VI | Collagène XII |
|---|---|---|
| | Col6A1 | Cola12A1 |
| Témoin | 100 | 100 |
| Rhamnose 1mM | 109 | 115 |
| Vitamine C 50µM | 154 | 124 |
| Rhamnose 1mM+Vitamine C 50µM | 174 | 152 |

Le tableau ci-dessus présente l'intensité de la biosynthèse des collagènes VI et XII dans les fibroblastes en présence de rhamnose, de vitamine C ou de leur association par rapport au témoin qui est à 100.

Les résultats sont également représentés sur la figure 5. Ils démontrent une synergie d'action du rhamnose et de la vitamine C dans la régulation de la transcription de gènes codant pour les collagènes de types VI et XII par les fibroblastes du derme.

### Exemple 6 : exemple de réalisation d'une composition cosmétique selon l'invention

| ***Crèmes régénération épidermiqe et dermique : émulsion huile dans eau*** | |
|---|---|
| Ammonium Polyacryldimethyltauramide (Hostacerin AMPS de Clariant) | 1,00% |
| Cyclohexasiloxane | 5,0% |
| Huile d'amande d'abricot | 7% |
| Isononyl isononanoate | 7% |
| Stearyl alcohol | 0,30% |
| Glyceryl stearate / PEG-100 stearate | 0,70% |
| Dimyristyl tartrate / cetearyl alcohol / C12-15 pareth-7 / PPG-25 laureth-25 | 0,50% |
| Gomme de Xanthan | 0,20 % |
| Rhamnose | 5% |
| Acide Ascorbique | 3% |
| Conservateurs | 0,50% |
| Eau | qsp 100 |

### Exemple 7 : exemple de réalisation d'une composition cosmétique selon l'invention

| ***Crèmes régénération épidermique : émulsion huile dans eau*** | |
|---|---|
| Ammonium Polyacryldimethyltauramide (Hostacerin AMPS de Clariant) | 1,00% |
| Cyclohexasiloxane | 5,0% |
| Glycerin | 1,70% |
| Stearyl alcohol | 0,30% |
| Huile d'amande d'abricot | 7% |
| Isononyl isononanoate | 7% |
| Dimyristyl tartrate / cetearyl alcohol / C12-15 pareth-7 / PPG-25 laureth-25 | 0,50% |
| Gomme de Xanthan | 0,20 % |
| Acide Ascorbique | 5% |
| SMA 1000 HNa | 1% |
| Mannose | 5% |
| Conservateurs | 0,50% |
| Eau | qsp 100 |

### Exemple 8 : exemple de réalisation d'une composition cosmétique selon l'invention

| ***Crèmes régénération épidermique : émulsion huile dans eau*** | |
|---|---|
| Ammonium Polyacryldimethyltauramide (Hostacerin AMPS de Clariant) | 1,00% |
| Cyclohexasiloxane | 5,0% |
| Glycerin | 1,70% |
| Stearyl alcohol | 0,30% |
| Huile d'amande d'abricot | 7% |
| Isononyl isononanoate | 7% |
| Dimyristyl tartrate / cetearyl alcohol / C12-15 pareth-7 / PPG-25 laureth-25 | 0,50% |
| Gomme de Xanthan | 0,20 % |
| Mannose | 2,5% |
| Rhamnose | 2,5% |
| Ascorbyl Phosphate de Mg | 1% |
| Conservateurs | 0,50% |
| Eau | qsp 100 |

### Exemple 9 : exemple de réalisation d'une composition cosmétique selon l'invention

### Crème de jour anti-âge pour le visage

### Phase A1 :-

- Distéarate de sucrose commercialisé par la Société STEARINERIE DUBOIS 1,75 %
- Stéarate de sorbitane oxyéthyléné à 4 moles d'oxyde d'éthylènecommercialisé par la Société ICI sous le nom "TWEEN 61" 1,15 %
- Acide stéarique 0,75 %
- Stearyl heptanoate 4,00 %
- Vaseline codex 1,50 %
- Huile d'avocat 3,20 %
- Huile de jojoba 3,00 %
- Huile de silicone volatile 2,70 %
- Acétate de vitamine E 1,00 %
- Glycérides de Vitamine F 3,00 %

### Phase A2 :

Gomme de silicone commercialisé par DOW CORNING sous le nom "Q2-1403 Fluid" 3,00 %
- Propylparaben 0,2 %
- Parfum 0,3 %

### Phase B

- Glycérine 3,00 %
- Hydroxyproline 1,00 %
- D-panthenol 1,00 %
- Triéthanolamine 0,35 %
- Rhamnose 3,00%
- Vit C glycosylé 1,00%
- Méthylparaben 0,3 %
- Eau déminéralisée q.s.p. 100 %

### Phase C :

- Ammonium Polyacryldimethyltauramide (Hostacerin AMPS de Clariant)1%

## Revendications

1. Utilisation cosmétique d'une composition comprenant, dans un milieu physiologiquement acceptable, l'association d'au moins un monosaccharide qu'est le rhamnose et d'au moins un composé additionnel choisi parmi l'acide ascorbique, un de ses analogues et leur mélange, dans laquelle l'analogue de l'acide ascorbique est choisi parmi l'ascorbate de sodium, l'ascorbylphosphate de magnésium ou de sodium, l'ester acétique de l'acide ascorbique, un ester d'ose de l'acide ascorbique, un sel métallique d'acide ascorbique phosphorylé et leur mélange, et dans laquelle la quantité dudit monosaccharide est comprise entre 0,4 % et 30 % en poids par rapport au poids total de la composition, pour diminuer et/ou prévenir les signes du vieillissement de la peau ou de ses phanères, lesdits les signes du vieillissement de la peau ou de ses phanères étant l'amélioration de la densité de la peau, sa fermeté et/ou de la cohésion de ses différents compartiments, en particulier de la cohésion du derme avec l'épiderme.

2. Utilisation selon la revendication 1, pour améliorer le profil lipidique des épidermes en modifiant la lipogenèse, et provoquer en particulier une augmentation de la synthèse des céramides, ou pour augmenter la synthèse de tenascine, de collagène VII, de collagène VI et/ou XII.

3. Utilisation selon la revendication 1 ou 2, dans laquelle le composé additionnel est l'acide ascorbique.

## Patentansprüche

1. Kosmetische Verwendung einer Zusammensetzung, umfassend in einem physiologisch verträglichen Medium die Kombination aus wenigstens einem Monosaccharid, das Rhamnose ist, und wenigstens einer zusätzlichen Verbindung, ausgewählt aus Ascorbinsäure, einem ihrer Analoga und Gemisch davon, wobei das Ascorbinsäure-Analogon aus Natriumascorbat, Magnesium- oder Natriumascorbylphosphat, Essigsäureascorbylester, einem Zuckerester der Ascorbinsäure, einem Metallsalz der phosphorylierten Ascorbinsäure und Gemisch davon ausgewählt ist, und wobei die Menge besagten Monosaccharids zwischen 0,4 Gew.-% und 30 Gew.-% bezogen auf das Gesamtgewicht der Zusammensetzung beträgt, zur Verringerung und/oder Verhinderung der Anzeichen der Alterung der Haut oder ihrer Hautanhangsgebilde, wobei die besagten Anzeichen der Alterung der Haut oder ihrer Hautanhangsgebilde die Verbesserung der Dichte der Haut, ihrer Festigkeit und/oder der Kohäsion ihrer verschiedenen Kompartimente, insbesondere der Kohäsion der Dermis mit der Epidermis, sind.

2. Verwendung gemäß Anspruch 1, zur Verbesserung des epidermalen Lipidprofils durch Modifizierung der Lipogenese und insbesondere zur Auslösung einer Erhöhung der Synthese von Ceramiden oder zur Erhöhung der Synthese von Tenascin, von Kollagen VII, von Kollagen VI und/oder XII.

3. Verwendung gemäß Anspruch 1 oder 2, wobei die zusätzliche Verbindung Ascorbinsäure ist.

## Claims

1. Cosmetic use of a composition comprising, in a physiologically acceptable medium, a combination of at least one monosaccharide that is rhamnose and at least one additional compound chosen from ascorbic acid, an analogue thereof and a mixture thereof, wherein the analogue of ascorbic acid is chosen from sodium ascorbate, magnesium or sodium ascorbyl phosphate, the acetic ester of ascorbic acid, a saccharide ester of ascorbic acid, a metal salt of phosphoryl ascorbic acid, and a mixture thereof, and in which the amount of the said monosaccharide is between 0.4% and 30% by weight relative to the total weight of the composition, for reducing and/or preventing the signs of ageing of the skin or its integuments, said signs of ageing of the skin or its integuments being improvement of the density of the skin, its firmness and/or the cohesion of its various compartments, in particular of the cohesion of the dermis with the epidermis.

2. Use according to claim 1, for improving the lipid profile of the epidermis by modifying lipogenesis, and in particular causing an increase in the synthesis of ceramides, or for increasing the synthesis of tenascin, collagen VII and collagen VI and/or XII.

3. Use according to claim 1 or 2, wherein the additional compound is ascorbic acid.
